## Europäisches Patentamt

(19) **European Patent Office**

**Office européen des brevets**

(11) Veröffentlichungsnummer: **0 259 492**

**A1**

(12) **EUROPÄISCHE PATENTANMELDUNG**

Veröffentlicht nach Art. 158 Abs. 3 EPÜ

(21) Anmeldenummer: **86902576.7**

(22) Anmeldetag: **30.01.86**

Daten der zugrundeliegenden internationalen Anmeldung:

(86) Internationale Anmeldenummer:
**PCT/SU86/00006**

(87) Internationale Veröffentlichungsnummer:
**WO87/04625 (13.08.87 87/18)**

(51) Int. Cl.³: **A 61 L 15/00**
**A 61 L 15/07**

(43) Veröffentlichungstag der Anmeldung:
**16.03.88 Patentblatt 88/11**

(84) Benannte Vertragsstaaten:
**BE CH DE FR GB IT LI**

(71) Anmelder: **VSESOJUZNY NAUCHNO-ISSLEDOVATELSKY I ISPYTATELNY INSTITUT MEDITSINSKOI TEKHNIKI**
**ul. Kasatkina, 3**
**Moscow, 129301(SU)**

(72) Erfinder: **SOLODOVNIK, Valentin Dmitrievich**
**ul. Novatorov, 36-9-15**
**Moscow, 117421(SU)**

(72) Erfinder: **LIR, Irina Lvovna**
**pr. Mira, 108-357**
**Moscow, 129626(SU)**

(72) Erfinder: **DAVYDOV, Anatoly Borisovich**
**ul. Krasny Kazanets, 19-1-283**
**Moscow, 111395(SU)**

(72) Erfinder: **SCHERBAK, Agnessa Sergeevna**
**Leningradskoe shosse, 41-2-37**
**Moscow, 125212(SU)**

(72) Erfinder: **LJUKEVICH, Igor Alexeevich**
**Trekhgorny val, 3-11**
**Moscow, 123022(SU)**

(72) Erfinder: **CHERKASHIN, Viktor Vasilievich**
**Leninsky pr., 44-224**
**Moscow, 117334(SU)**

(74) Vertreter: **von Füner, Alexander, Dr. et al,**
**Patentanwälte v. Füner, Ebbinghaus, Finck Mariahilfplatz 2 & 3**
**D-8000 München 90(DE)**

(54) **POLYURETHANSCHAUMZUBEREITUNG FÜR TRAUMATOLOGISCHE IMMOBILISIERUNGEN.**

(57) Schaumpolyurethan-Mischung zur Immobilisierung in der Traumatologie, die hydroxylhaltige einen Katalysator, ein Aufschäumungsmittel, ein Schaumstabilisierungsmittel und Polyisozyanat in einer Menge enthält, die ein Verhältnis der Isozyanatgruppen zu den Hydroxylgruppen des Polyethers von 1:1 gewährleistet, dadurch gekennzeichnet, daß sie zusätzlich ein niedermolekulares Polyolefin bei folgendem Verhältnis der Ausgangskomponenten in Masse-% enthält:

| | |
|---|---|
| hydroxylhaltiger Polyether | 20 bis 30 |
| Katalysator | 0,5 bis 3,0 |
| Aufschäumungsmittel | 0,5 bis 7,0 |
| Schaumstabilisierungsmittel | 0,5 bis 0,9 |
| niedermolekulares Polyolefin | 20 bis 33 |
| Polyisozyanat, das ein Verhältnis der Isozyanatgruppen zu den Hydroxylgruppen des Polyethers 1:1 gewährleistet | Rest. |

./...

Croydon Printing Company Ltd

EP 0 259 492 A1

Schaumpolyurethan-Mischung nach Anspruch 1, dadurch gekennzeichnet, daß sie als niedermolekulares Polyolefin Vaselinöl beziehungsweise Hartparaffin enthält.

Schaumpolyurethan-Mischung nach Anspruch 1 und 2, dadurch gekennzeichnet, daß sie als hydroxylhaltiges Polyether ein Produkt der Hydroxyalkylierung eines mehrwertigen Alkohols mit Hydroxyalkylen enthält.

Schaumpolyurethan-Mischung nach Anspruch 1 bis 3, dadurch gekennzeichnet, daß sie als Katalysator hydroxylhaltiges tertiäres Amin enthält.

Schaumpolyurethan-Mischung nach Anspruch 1 bis 4, dadurch gekennzeichnet, daß sie als Treibmittel Trichlorfluormethan enthält.

Schaumpolyurethan-Mischung nach Anspruch 1 bis 5, dadurch gekennzeichnet, daß sie als Schaumstabilisierungsmittel siliziumorganische Oligomere beziehungsweise Produkte ihrer Hydroxyalkylierung enthält.

Schaumpolyurethan-Mischung nach Anspruch 1 bis 6, dadurch gekennzeichnet, daß sie als Polyisozyanat 4,4-Diphenylmethandiisozyanat beziehungsweise sein Gemisch mit aromatischen Isozyanaten enthält.

## SCHAUMPOLYURETHAN-MISCHUNG ZUR IMMOBILISIERUNG IN DER TRAUMATOLOGIE

### Gebiet der Technik

Die vorliegende Erfindung bezieht sich auf die Medizin, und betrifft insbesondere eine Schaumpolyurethan-Mischung zur Immobilisation in der Traumatologie.

### Vorhergehender Stand der Technik

Bekannt ist eine Mischung für die Herstellung eines immobilisierenden Schaumstoffes auf der Grundlage von Polyurethan (US, A, 2947307, 1960, Kl. 128-90), die Polyester mit Hydroxylgruppen, Hexamethylendiisozyanat, tertiäres Amin als Katalysator und Wasser als Aufschäumungsmittel enthält. Die genannte Mischung wird durch eine hohe Viskosität des Polyesters, was seine Verwendung unter den nichtstationären Bedingungen erschwert beziehungsweise unmöglich macht, sowie durch eine hohe Wärmeentwicklung gekennzeichnet, die auf eine hohe Aktivität primärer Hydroxylgruppen in Polyestern zurückzuführen ist. Die hohe Wärmeentwicklung führt zur Notwendigkeit, Polyester mit geringer Anzahl von Hydroxylgruppen zwecks Vermeidung von Brandverletzungen zu verwenden, und das führt seinerseits zur Senkung der Härte von Schaumpolyurethan.

Bekannt ist ebenfalls eine Mischung (US, A, 3301252, 1967, Kl. 128-90) zur Herstellung eines harten immobilisierenden Schaumstoffes, die einen aromatischen Polyether aufweist, der ein Produkt der Kondensation von 1,1,3-Hydroxyphenylpropan und von Propylenoxid mit einer Hydroxylzahl von etwa 380, Polyphenylmethanpolyisozyanat, darstellt, das von 40 bis 60 Gew.-% 4,4-Diphenylmethandiisozyanat, Trichlorfluormethan als Aufschäumungsmittel, 1-Methyl-4-dimethylaminoethylpiperazin als Katalysator und ein Blockmischpolymer des Siloxans und des Hydroxyalkens als Schaumstabilisierungsmittel enthält.

Zum Nachteil dieser Mischung gehört eine hohe Viskosität des aromatischen Polyethers, eine hohe Temperatur

- 2 -

der Bildung des Schaumpolyurethans und die Toxizität des Katalysators.

Bekannt ist auch eine Mischung (SU, A, 433181, 1971, Kl. C 08 G 18/14), die als hydroxylhaltige Verbindung ein Gemisch (1:9 - 4:6) des N, N, N', N'-Tetraoxypropylethylendiamins und des Polyethers auf der Grundlage von Xylit, Polyisozyanat und ein Aufschäumungsmittel aufweist.

Zum Nachteil dieser Mischung gehört auch eine hohe Temperatur der Bildung von Schaumpolyurethan.

Bekannt ist ebenfalls eine Mischung (SU, A,990213, 1975, Kl. A 61 F 1/00), die folgende Komponenten (Masse%) aufweist:

| | |
|---|---|
| Aufschäumungsmittel | 1 bis 40 |
| hydroxylhaltiger Polyether | 25 bis 45 |
| polyhydroxylhaltiges tertiäres Amin | 1,6 bis 14 |
| monohydroxylhaltiges tertiäres Amin | 0,16 bis 2,3 |
| tertiäres Amin, das keine Hydroxylgruppen aufweist | 0,16 bis 3,2 |
| Polyisozyanat in einer Menge, die ein Verhältnis der Isozyanatgruppen zu den Hydroxylgruppen des Polyethers 1:1 gewährleistet | Rest. |

Als hydroxylhaltiger Polyether werden Produkte der Hydroxyalkylierung mehrwertiger Alkohole und als Schaumstabilisierungsmittel eine siliziumorganische Verbindung verwendet. Die Mischung gewährleistet eine erforderliche Geschwindigkeit der Bildung des Schaumpolyurethans und seine Härtung bei erniedrigter Temperatur; unter den gewöhnlichen Bedingungen erfordert jedoch die hohe Wärmeentwicklung die Verwendung wärmeisolierender Dichtungen zwecks Vermeidung der möglichen Brandverletzung des Körpers eines Patienten.

## Offenbarung der Erfindung

Der Erfindung liegt die Aufgabe zugrunde, durch

- 3 -

Einführung einer neuen Komponente und Änderung des mengenmäßigen Verhältnisses der Ausgangskomponenten eine Schaumpolyurethan-Mischung herzustellen, die eine niedrige Temperatur der Schaumbildung, eine verbesserte Wasserbeständigkeit aufweist, die bei ihrer Anwendung die Möglichkeit der Brandverletzungen eines Patienten ausschließt.

Die Aufgabe wird dadurch gelöst, daß die erfindungsgemäße Schaumpolyurethan-Mischung zur Immobilisierung in der Traumatologie, die ein hydroxylhaltiges Polyether, einen Katalysator, ein Aufschäumungsmittel, ein Schaumstabilisierungsmittel, Polyisozyanat in einer Menge aufweist, die ein Verhältnis der Isozyanatgruppen zu den Hydroxylgruppen des Polyethers von 1:1 gewährleistet, erfindungsgemäß, zusätzlich niedermolekulares Polyolefin bei folgendem Verhältnis der Ausgangskomponenten, in Masse% enthält:

| | |
|---|---|
| hydroxylhaltiger Polyether | 20 bis 30 |
| Katalysator | 0,5 bis 3,0 |
| Aufschäumungsmittel | 0,5 bis 7,0 |
| Schaumstabilisierungsmittel | 0,5 bis 0,9 |
| niedermolekulares Polyolefin | 20 bis 33 |
| Polyisozyanat, das ein Verhältnis der Isozyanatgruppen zu den Hydroxylgruppen des Polyethers 1:1 gewährleistet | Rest. |

Die Einführung in die Zusammensetzung der Mischung des niedermolekularen Polyolefins in einer Menge von 20 bis 30 Masse% verringert die Wärmeentwicklung bei der Aushärtung des Schaumstoffes.

Als niedermolekulares Polyolefin weist die genannte Mischung vorzugsweise Vaselinöl beziehungsweise Hartparaffin auf, als hydroxylhaltiges Polyether enthält das Produkt der Hydroxyalkylierung des mehrwertigen Alkohols mit Hydroxyalkylen.

Die erfindungsgemäße Mischung weist als Katalysator vorzugsweise hydroxylhaltiges tertiäres Amin, als Aufschäumungsmittel Trichlorfluormethan, als Schaumsta-

- 4 -

bilisierungsmittel siliziumorganische Oligomere beziehungsweise Produkte ihrer Hydroxyalkylierung und als Polyisozyanat das 4,4-Diphenylmethandiisozyanat beziehungsweise sein Gemisch mit aromatischen Isozyanaten auf. Das bewirkt die Herstellung eines leichten, röntgentransparenten, nichttoxischen und festen Materials aus der genannten Mischung.

Die erfindungsgemäße Mischung weist im Vergleich zu den bekannten Mischungen eine niedrigere Temperatur der Schaumbildung und eine verbesserte Wasserbeständigkeit auf.

### Beste Ausführungsvariante der Erfindung

Die erfindungsgemäße Schaumpolyurethan-Mischung zur Immobilisation in der Traumathologie weist folgende Zusammensetzung in Masse% auf:

| | |
|---|---|
| hydroxylhaltiger Polyether | 20 bis 30 |
| Katalysator | 0,5 bis 3,0 |
| Aufschäumungsmittel | 0,5 bis 7,0 |
| Schaumstabilisierungsmittel | 0,5 bis 0,9 |
| niedermolekulares Polyolefin | 20 bis 33 |
| Polyisozyanat, das ein Verhältnis der Isozyanatgruppen zu den Hydroxylgruppen des Polyethers 1:1 gewährleistet | Rest. |

Als niedermolekulare Polyolefine können flüssige und feste Polyolefine, beispielsweise, Mineralöl, vorzugsweise Vaselinöl, Silikonflüssigkeiten und feste Paraffine mineralischer und synthetischer Herkunft mit einer Molekularmasse von 300 bis 2000, vorzugsweise feste Paraffine mit einem Schmelzpunkt von 30 bis 80°C, beispielsweise, Polyethylenwachs beziehungsweise Paraffin, sowie andere gegenüber den Isozyanaten und Alkoholen inerte hochsiedende organische Flüssigkeiten beziehungsweise feste Stoffe mit analogem Schmelzpunkt dienen.

Die Eigenschaften des Stoffes werden durch die Menge des eingeführten niedermolekularen Polyolefins bestimmt, das eine wärmeentziehende Komponente darstellt,

- 5 -

deren Gehalt 20 bis 30 Gew.-% beträgt. Die Einführung der genannten Komponente in einer Menge unter 20 Gew.-% führt zur Steigerung der Temperatur der Schaumbildung bis zu den Werten, die 45°C übersteigen, was die Gefahr der einer Brandverletzung der Haut eines Patienten schafft. Die Einführung des wärmeentziehenden Mittels in einer Menge über 30 Gew.-% führt schon nicht zu einer wesentlichen Steigerung der Temperatur der Aushärtung und ruft die Ausscheidung von Paraffin in eine selbständige Phase hervor.

Als hydroxylhaltige Polyether können aliphatische, alizyklische beziehungsweise aromatische Polyether mit einer Funktionalität von 3 bis 8 verwendet werden, die von 8 bis 22 Gew.-% Hydroxylgruppen enthalten sowie Aminogruppen aufweisen, beispielsweise ein Produkt der Hydroxypropylierung eines mehrwertigen Alkohols (Glyzerin, Xylit beziehungsweise Saccharose), stickstoffhaltiges Polyether N, N, N', N'-Tetraoxypropylethylendiamin beziehungsweise ihr Gemisch in einem Verhältnis von 5-30:95-70.

Als hydroxylhaltiges Polyether können auch Polyester, beispielsweise, Prokukte der Kondensation aliphatischer Dikarbonsäuren (Adipin-, Phthalsäuren) und mehrwertiger Alkohole (Butylenglykol, Hexantriol) verwendet werden, die gegenüber den Säuren im Überschuß genommen werden.

Als Polyisozyanat kann man aliphatische und aromatische Polyisozyanate mit einer Funktionalität 2 und darüber hinaus sowie Produkte ihrer Umsetzung mit Polyestern, vorzugsweise 4,4-Diphenylmethandiisozyanat beziehungsweise sein Gemisch mit aromatischen Isozyanaten mit größerer Funktionalität verwenden.

Als Katalysatoren können tertiäre Amine, zinnorganische Verbindungen und andere Stoffe, die die Bildung von Urethanen aus den Isozyanaten fördern, beispielsweise N, N-Dimethylethanolamin, N, N-Diethylethanolamin, Triethylamin, Dimethylcyklohexylamin beziehungsweise ihr

- 6 -

Gemisch, vorzugsweise N, N-Dimethylethanolamin verwendet werden.

Das Vorhandensein tertiärer Amine der Hydroxylgruppen in einem Molekül bewirkt ihre chemische Bindung mit einem Polyisozyanat und hierdurch die Verringerung der Toxizität des Schaumstoffes.

Als Aufschäumungsmittel können niedermolekulare fluorierte Kohlenwasserstoffe, Chlorkohlenwasserstoffe, vorzugsweise Trichlorfluormethan und ihre Gemische, andere niedersiedende inerte organische Flüssigkeiten sowie Wasser dienen.

Als Schaumstabilisierungsmittel können siliziumorganische Oligomere und Produkte ihrer Hydroxyalkylierung verwendet werden.

Das Aufschäumen, das Formen und die Aushärtung der Mischung erfolgen bei Raumtemperatur nach folgender Methode: in einem Behälter bringt man die berechneten Mengen aller Komponenten außer dem Polyisozyanat unter und mischt sie; dann setzt man dem Gemisch das Polyisozyanat zu, mischt während 30 Sekunden und läßt zum freien Aufschäumen bis zum Erreichen des Zeitpunktes des Klebens stehen. Dann wird die aufgeschäumte Masse auf den zu immobilisierenden Körperabschnitt eines Verletzten aufgetragen, in einer gleichmäßigen Schicht vertrieben und bis zur Aushärtung stehengelassen.

Der ausgehärtete Schaumstoff erhält die ihm verliehene Form während einer unbestimmt langen Zeit, läßt sich nicht zerstören, verletzt nicht die Haut des Patienten, läßt sich leicht mit einem Messer beziehungsweise mit einer Gipsschere schneiden.

Die erfindungsgemäße Mischung würde in einer Klinik an 23 Patienten erprobt. In 7 Fällen wurden Fixierlangetten im Gießverfahren und in 16 Fällen Fixiervorrichtungen aus dem Schaumstoff unter Einsatz von Konturen--Polyethylenüberzügen gefertigt, die zweiwändige Überzüge darstellen, die nach der Form der jeweiligen Gliedmaßen zugeschnitten werden.

Gefertigt werden folgende Typen von fixierenden Vorrichtungen: Langetten für obere Extremität - 7, Langetten für die Fixierung des Unterschenkels und des Fusses - 11, Schienen zur Fixierung des Kniegelenkes - 3, Korsette zur Fixierung der Lendenwirbelsäule - 1 und orthopädische Schanzsche Krawatte zur Fixierung der Halswirbelsäule - 1.

Nach Angaben der klinischen Beobachtungen ruft der Schaumstoff beim Anlegen keine Hyperämie beziehungsweise keine Reizungen hervor, seine Erstarrung erfolgt innerhalb von 8 bis 10 Minuten nach dem Vermischen der Komponenten. Bei der Aufschäumung modelliert der Stoff gut die Form einer Extremität beziehungsweise eines Körperteils nach. Der erstarrte Schaumstoff der erfindungsgemäßen Zusammensetzung weist eine hohe Festigkeit, Wasserbeständigkeit auf, ist röntgentransparent, leicht, seine immobilisierenden Eigenschaften bleiben während einer unbestimmt langen Zeit aufrechterhalten, zugleich läßt sich der Stoff leicht mit einem Messer schneiden.

Fixierende Elemente aus dem Schaumstoff der erfindungsgemäßen Zusammensetzung können zur Immobilisierung beim Transport und bei der Behandlung in den Fällen verwendet werden, in denen übelicherweise Gipsimmobilisierung verwendet wird.

Hierdurch weist die erfindungsgemäße Mischung im Vergleich zu den bekannten Mischungen wesentliche Vorteile auf: Leichtigkeit, Röntgentransparenz, Wasserbeständigkeit, niedrige Temperatur der Erstarrung (Ausschließung der Möglichkeit von Brandverletzungen der Haut).

Zur besseren Erläuterung der vorliegenden Erfindung werden konkrete Varianten der erfindungsgemäßen Mischung angeführt.

Beispiel 1

Schaumpolyurethan-Mischung zur Immobilisierung in der Traumatologie folgender Zusammensetzung (in Masse%):

| | |
|---|---|
| Produkt der Hydroxypropylierung von Xylit mit einer Molekularmasse von 800 | 16,6 |
| Produkt der Hydroxyethylierung von Glyzerin mit einer Molekularmasse von 350 | 3,4 |
| N,N -Dimethylethanolamin | 0,5 |
| Trichlorfluormethan | 0,5 |
| Produkt der Hydroxyethylierung des Dihydroxypolydimethylsiloxans mit einer Molekularmasse von 400 | 0,5 |
| Paraffin mit einem Schmelzpunkt von 39 bis 40°C | 33,0 |
| Gemisch (1:5) aus 4,4-Diphenylmethan-diisozyanat mit den Polyisozyanaten allgemeiner Formel OC $NC_6H_4CH_2[C_6H_3(NCO)CH_2]_n C_6H_4NCO$ | 45,5 |

worin n = 1 bzw. 2 ist.

In einen Behälter aus Polyethylen mit einem Inhalt von 200 ml werden die genannten Mengen aller Komponenten, außer dem Gemisch der Polyisozyanate, eingebracht, sie werden vermischt; dann setzt man dem hergestellten Gemisch ein Gemisch der Polyisozyanate hinzu und vermischt es während 30 Sekunden, wonach man es zum freien Aufschäumen bis zum Erreichen des Zeitpunktes des Klebens stehenläßt. Dann wird die aufgeschäumte Masse geformt und ausgehärtet.

Die maximale Temperatur zum Zeitpunkt der Aufschäumung der Mischung übersteigt nicht 45°C, dann sinkt sie bis zur Raumtemperatur. Die scheinbare Dichte des Schaumstoffes beträgt 40 kp/m$^3$. Beim Auflegen des Schaumstoffes auf Gliedmaßen eines Patienten (während der klinischen Versuche) wurden keine Reizungen, keine Hyperämie oder Brandverletzung der Haut eines Patienten nachgewiesen.

Beispiel 2

Schaumpolyurethan-Mischung zur Immobilisierung in der Traumathologie folgender Zusammensetzung (in Masse%):

Produkt der Hydroxypropylierung von
Glyzerin mit einer Molekularmasse von 400          10,0

Produkt der Hydroxyethylierung von Xylit
mit einer Molekularmasse von 800                   20,0

N,N -Diethylethanolamin                             3,0

Trichlorfluormethan                                 7,0

Produkt der Hydroxyethylierung von
Dihydroxypolydimethylsiloxan mit
einer Molekularmasse von 400                        0,9

Vaselinöl mit einer Molekularmasse von 350         20,0

Gemisch (1:1) des 4,4-Diphenylmethandi-
isozyanats und der Polyisozyanate allgemeiner Formel
$OCNC_6H_4CH_2[C_6H_3(NCO)CH_2]_n C_6H_4NCO$      39,1 ,

worin n = 1 bzw. 2 ist.

Die Mischung schäumt man auf, formt und härtet aus wie in Beispiel 1 beschrieben. Die Temperatur der Aufschäumung der Mischung beträgt von 44 bis 45°C, die scheinbare Dichte beträgt 41 kp/m$^3$. Bei der klinischen Erprobung wurde keine ungünstige Einwirkung des Schaumstoffes auf die Haut eines Patienten nachgewiesen.

Beispiel 3

Schaumpolyurethan-Mischung zur Immobilisierung in der Traumatologie folgender Zusammensetzung (in Masse%):

Produkt der Hydroxypropylierung von
Xylit mit einer Molekularmasse von 800             20,75

Produkt der Hydroxyethylierung von Glyzerin mit einer Molekularmasse von 350               4,25

N,N -Dimethylethanolamin                            1,50

Trichlorfluormethan                                 5,5

- 10 -

Produkt der Hydroxyethylierung von
Dihydroxypolydimethylsiloxan mit
einer Molekularmasse von 400                              0,7

Paraffin mit einem Schmelzpunkt von
35 bis 40°C                                              30,3

Gemisch der Polyisozyanate (ähnlich
wie in Beispiel 2 beschrieben)                           37,0 .

Die Mischung schäumt man auf, formt und härtet aus wie in Beispiel 1 beschrieben. Die maximale Temperatur der Aufschäumung beträgt von 44 bis 45°C, die scheinbare Dichte beträgt 40 kp/m$^3$. Bei der klinischen Erprobung wurde keine ungünstige Einwirkung des Schaumstoffes auf die Haut eines Patienten nachgewiesen.

Beispiel 4

Schaumpolyurethan-Mischung zur Immobilisierung in der Traumatologie folgender Zusammensetzung (in Masse%):

Produkt der Hydroxypropylierung von Xylit mit
einer Molekularmasse von 800                             20,75

Produkt der Hydroxyethylierung von Glyzerin mit einer Molekularmasse von 350                      4,25

N, N -Dimethylethanolamin                                 1,5

Trichlorfluormethan                                       5,5

Produkt der Hydroxyethylierung von
Dihydroxypolydimethylsiloxan mit
einer Molekularmasse von 400                              0,7

Paraffin mit einem Schmelzpunkt von
35 bis 40°C                                              30,3

4,4-Diphenylmethandiisozyanat                            37,0 .

Die Mischung schäumt man, formt und härtet ähnlich wie in Beispiel 1 beschrieben aus.

Die maximale Temperatur der Aufschäumung beträgt von 44 bis 45°C, die scheinbare Dichte beträgt 40 kp/m$^3$. Bei der klinischen Erprobung wurde keine ungünstige Einwirkung des Schaumstoffes auf die Haut eines Patienten nachgewiesen.

### Beispiel 5 (zur Kontrolle)

Schaumpolyurethan-Mischung zur Immobilisierung in der Traumatologie folgender Zusammensetzung (in Masse%):

| | |
|---|---|
| Produkt der Hydroxypropylierung von Xylit mit einer Molekularmasse von 800 | 29,76 |
| Produkt der Hydroxyethylierung von Glyzerin mit einer Molekularmasse von 350 | 6,09 |
| N, N - Dimethylethanolamin | 2,15 |
| Trichlorfluormethan | 7,88 |
| Produkt der Hydroxyethylierung von Dihydroxypolydimethylsiloxan mit einer Molekularmasse von 400 | 1,04 |
| Gemisch (1:1) aus 4,4-Diphenylmethandiisozyanat mit den Polyisozyanaten allgemeiner Formel $OCNC_6H_4CH_2[C_6H_3(NCO)CH_2]_n C_6H_4NCO$ | 53,08 , |

worin n = 1 bzw. 2 ist.

Die Mischung schäumt man auf, formt und härtet aus wie in Beispiel 1 beschrieben.

Die maximale Temperatur der Aufschäumung beträgt 109°C, die scheinbare Dichte beträgt 17 kp/m$^3$. Beim Auflegen auf die Haut von Ratten, die von der Haardecke befreit ist, wurde die Brandverletzung (Combustio bullosa und Combustio gangraenosa) beobachtet.

### Industrielle Anwendbarkeit

Die erfindungsgemäße Polyurethan-Mischung kann als Immobilisierungsstoff bei der medizinischen Hilfe bei Knochenbruch, Verwundungen und anderen Verletzungen von Gliedmaßen unter stationären und nichtstationären Bedingungen eingesetzt werden.

PATENTANSPRÜCHE:

1. Schaumpolyurethan-Mischung zur Immobilisierung in der Traumatologie, die hydroxylhaltige einen Katalysator, ein Aufschäumungsmittel, ein Schaumstabilisierungsmittel und Polyisozyanat in einer Menge enthält, die ein Verhältnis der Isozyanatgruppen zu den Hydroxylgruppen des Polyethers von 1:1 gewährleistet, d a d u r c h g e k e n n z e i c h n e t , daß sie zusätzlich ein niedermolekulares Polyolefin bei folgendem Verhältnis der Ausgangskomponenten in Masse-% enthält:

| | |
|---|---|
| hydroxylhaltiger Polyether | 20 bis 30 |
| Katalysator | 0,5 bis 3,0 |
| Aufschäumungsmittel | 0,5 bis 7,0 |
| Schaumstabilisierungsmittel | 0,5 bis 0,9 |
| niedermolekulares Polyolefin | 20 bis 33 |
| Polyisozyanat, das ein Verhältnis der Isozyanatgruppen zu den Hydroxylgruppen des Polyethers 1:1 gewährleistet | Rest. |

2. Schaumpolyurethan-Mischung nach Anspruch 1, d a d u r c h g e k e n n z e i c h n e t , daß sie als niedermolekulares Polyolefin Vaselinöl beziehungsweise Hartparaffin enthält.

3. Schaumpolyurethan-Mischung nach Anspruch 1 und 2, d a d u r c h g e k e n n z e i c h n e t , daß sie als hydroxylhaltiges Polyether ein Produkt der Hydroxyalkylierung eines mehrwertigen Alkohols mit Hydroxyalkylen enthält.

4. Schaumpolyurethan-Mischung nach Anspruch 1 bis 3, d a d u r c h g e k e n n z e i c h n e t , daß sie als Katalysator hydroxylhaltiges tertiäres Amin enthält.

5. Schaumpolyurethan-Mischung nach Anspruch 1 bis 4, d a d u r c h g e k e n n z e i c h n e t , daß sie als Treibmittel Trichlorfluormethan enthält.

6. Schaumpolyurethan-Mischung nach Anspruch 1 bis 5, dadurch gekennzeichnet, daß sie als Schaumstabilisierungsmittel siliziumorganische Oligomere beziehungsweise Produkte ihrer Hydroxyalkylierung enthält.

7. Schaumpolyurethan-Mischung nach Anspruch 1 bis 6, dadurch gekennzeichnet, daß sie als Polyisozyanat 4,4-Diphenylmethandiisozyanat beziehungsweise sein Gemisch mit aromatischen Isozyanaten enthält.

0259492

# INTERNATIONAL SEARCH REPORT

International Application No   PCT/SU86/00006

## I. CLASSIFICATION OF SUBJECT MATTER (if several classification symbols apply, indicate all)

According to International Patent Classification (IPC) or to both National Classification and IPC

IPC⁴ - A 61 L 15/00, 15/07

## II. FIELDS SEARCHED

### Minimum Documentation Searched

| Classification System | Classification Symbols |
|---|---|
| IPC⁴ | A 61 L 15/00, 15/07, A 61 B 17/56 |

### Documentation Searched other than Minimum Documentation to the Extent that such Documents are Included in the Fields Searched

## III. DOCUMENTS CONSIDERED TO BE RELEVANT

| Category | Citation of Document, with indication, where appropriate, of the relevant passages | Relevant to Claim No. |
|---|---|---|
| A | SU,A1,990213(V.G.Vakhtin et al.)28 January 1983(28.01.83),see the claims | 1 |
| A | US,A,3674901,(National Patent Development Corporation),04 July 1972(04.07.72), see the abstract | 1 |
| A | US,A,4203435(Firma Carl Freudenberg),20 May 1980(20.05.80),see the abstract | 1 |
| A | FR,A1,2425863(JOHNSON & JOHNSON)14 December 1979(14.12.79),see the abstract | 1 |

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art.
"&" document member of the same patent family

## IV. CERTIFICATION

| Date of the Actual Completion of the International Search | Date of Mailing of this International Search Report |
|---|---|
| 10 September 1986(10.09.86) | 21 October 1986(21.10.86) |

| International Searching Authority | Signature of Authorized Officer |
|---|---|
| ISA/SU | |

Form PCT/ISA/210 (second sheet) (January 1985)